(19) Europäisches Patentamt
European Patent Office
Office européen des brevets

(11) **EP 3 581 099 A1**

(12) **EUROPEAN PATENT APPLICATION**

(43) Date of publication:
**18.12.2019 Bulletin 2019/51**

(21) Application number: **18176971.2**

(22) Date of filing: **11.06.2018**

(51) Int Cl.:
**A61B 5/0205** (2006.01)    **A61B 5/024** (2006.01)
**A61B 5/0245** (2006.01)    **A61B 5/029** (2006.01)
**A61B 5/053** (2006.01)    **A61B 5/11** (2006.01)
**A61B 5/22** (2006.01)    **A61B 5/00** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Polar Electro Oy**
**90440 Kempele (FI)**

(72) Inventors:
• **Olstad, Daniela**
**90440 Kempele (FI)**

• **Korhonen, Topi**
**90440 Kempele (FI)**
• **Korkala, Seppo**
**90440 Kempele (FI)**
• **Hartikainen, Tuomas**
**90440 Kempele (FI)**
• **Kemppainen, Juhani**
**90440 Kempele (FI)**
• **Ahola, Riikka**
**90440 Kempele (FI)**

(74) Representative: **Kolster Oy Ab**
**(Salmisaarenaukio 1)**
**P.O. Box 204**
**00181 Helsinki (FI)**

(54) **STROKE VOLUME MEASUREMENTS IN TRAINING GUIDANCE**

(57)    The present document discloses a computer-implemented method for providing training guidance in connection with a physical exercise, comprising: measuring, by using a heart activity sensor, heart activity of a user during a physical exercise; measuring, by using a bioimpedance measurement sensor, bioimpedance of the user during the physical exercise; computing, by a processing circuitry, a stroke volume from the bioimpedance synchronized to a cardiac cycle of the user by using the measured heart activity; computing, by the processing circuitry, training intensity by using at least the computed the stroke volume; comparing, by the processing circuitry, the training intensity with at least one threshold and outputting at least one training guidance instruction on the basis of the comparison.

START

300: MEASURE HEART ACTIVITY AND BIOIMPEDANCE DURING PHYSICAL EXERCISE

302: COMPUTE STROKE VOLUME FROM BIOIMPEDANCE IN SYNC WITH CARDIAC CYCLE

304: COMPUTE TRAINING INTENSITY FROM STROKE VOLUME

306: COMPARE TRAINING INTENSITY WITH AT LEAST ONE THRESHOLD

308: THRESHOLD EXCEEDED?    NO    YES

310: OUTPUT TRAINING GUIDANCE INSTRUCTION(S)

Fig 3

EP 3 581 099 A1

**Description**

TECHNICAL FIELD

The present invention relates to a field of physiological or biometric measurements and, in particular, to measuring heart stroke volume by using bioimpedance measurements.

TECHNICAL BACKGROUND

A typical configuration for measuring bioimpedance includes a set of measurement electrodes disposable to contact with skin, a measurement circuitry for measuring bioimpedance from one or more of the electrodes, and a processing circuitry for processing measurement data. There may also be provided a communication circuitry for communicating the processed measurement data in a wired or wireless manner. Bioimpedance measurements enable detection of various physiological characteristics from a user performing a physical exercise.

BRIEF DESCRIPTION

The present invention is defined by the subject matter of the independent claims.
Embodiments are defined in the dependent claims.

BRIEF DESCRIPTION OF THE DRAWINGS

In the following the invention will be described in greater detail by means of preferred embodiments with reference to the attached [accompanying] drawings, in which

Figure 1 illustrates a garment comprising a measurement circuitry according to an embodiment;
Figure 2 an embodiment of the measurement circuitry;
Figure 3 illustrates a process for providing training guidance according to an embodiment of the invention;
Figure 4 illustrates an embodiment of stroke-volume-based training intensity zones;
Figure 5 illustrates an embodiment of an arrangement for switching a mode of one or more electrodes of the garment;
Figure 6 illustrates a flow diagram of a process for configuring the operation of the electrodes of the garment according to an embodiment of the invention;
Figure 7 illustrates an embodiment for accumulating stroke-volume-based training intensity during the exercise;
Figure 8 illustrates characteristics of the stroke volume and heart rate during the exercise;
Figure 9 illustrates a process for adapting duration of a rest period of an interval exercise according to an embodiment of the invention;
Figure 10 illustrates a process for adapting duration of a work period of an interval exercise according to an embodiment of the invention;
Figure 11 illustrates an example of guidance during a physical exercise when employing the processes of Figures 9 and 10;
Figure 12 illustrates a process for monitoring users fatigue level during the exercise according to an embodiment of the invention;
Figures 13 and 14 illustrate some embodiments of a detachable measurement circuitry attachable to the garment of Figure 1;
Figure 15 illustrates a process for monitoring user's dehydration state during the exercise according to an embodiment of the invention;
Figure 16 illustrates a process for estimating stroke volume when stroke volume measurement data is not available;
Figure 17 illustrates a process for estimating a performance index on the basis of measured stroke volume; and
Figure 18 illustrates a block diagram of an apparatus according to an embodiment of the invention.

DETAILED DESCRIPTION OF THE INVENTION

The following embodiments are exemplifying. Although the specification may refer to "an", "one", or "some" embodiment(s) in several locations of the text, this does not necessarily mean that each reference is made to the same embodiment(s), or that a particular feature only applies to a single embodiment. Single features of different embodiments may also be combined to provide other embodiments.

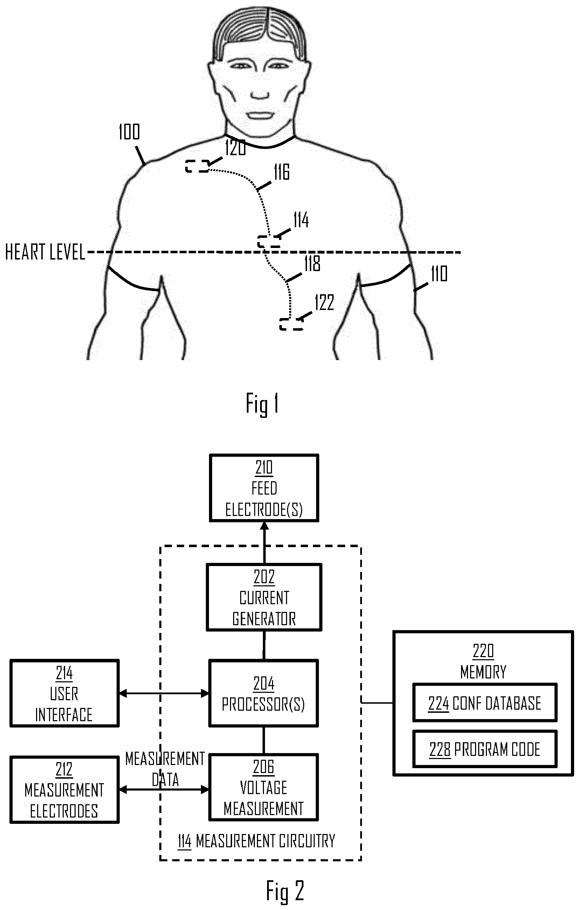

Figure 1 illustrates an embodiment of a garment 100 made of flexible material. The garment comprises: a first measurement electrode 120 integrated into the garment at a first location, a second measurement electrode 122 integrated into the garment at a second location different from the first location, and a measurement circuitry 114 configured to measure bioimpedance by using the first measurement electrode and the second electrode and to transmit the measured bioimpedance, and to measure electrocardiogram (ECG) by using at least one of the first measurement electrode and the second electrode or another electrode, wherein the garment is an upper body garment, wherein the first measurement electrode is disposed above a heart level and the second measurement electrode is disposed below the heart level.

In an embodiment, the garment is a shirt, a vest, or a harness. The garment may equally be a bra or any other garment designed as an under layer to contact the skin of a human 110 (a user). The garment may be made of one or more of the following materials: nylon, polyamide, elastane, polyester, cotton, and wool.

In the embodiment of Figure 1, the first measurement electrode 120 is disposed above the heart level, when the garment is worn by the user 110, while the second measurement electrode 122 is disposed below the heart level. The measurement circuitry 114 may be provided in a casing at an arbitrary location in the garment, and signal lines 116, 118 may connect the measurement electrodes 120, 122 to the measurement circuitry 114. The signal lines may be integrated into the garment.

Let us now describe the structure of the measurement circuitry 114 in greater detail with reference to Figure 2 illustrating an embodiment of an apparatus. The apparatus may comprise at least one processor 204 and at least one memory 220 storing a computer program 228 comprising a program instructions that configure the at least one processor to execute the embodiments described herein. The apparatus may further comprise a user interface 214 comprising at least a display device and a user input device.

The bioimpedance measurement may be carried out by arranging signal feed electrodes 210 in the garment and, further arranging measurement the electrodes 212 in the garment. The measurement electrodes 212 may comprise the electrodes 120, 122. The measurement circuitry 114 may be configured to control the signal feed and the measurement, e.g. the following manner. One or more processors 204 may control a current generator 202 to output electric current to the feed electrodes 210. The current generator 202 may be signal synthesizer capable of outputting alternating current one various frequencies. The biompedance measurements may be used for estimating body composition, and multiple frequencies may be output for that purpose.

While the current generator is outputting current to the body, the processor 204 may configure a voltage measurement circuitry 206 to measure voltage between the measurement electrodes 212 and to acquire voltage measurement data from the measurement electrodes. The knowledge of the measured voltage U and the applied current I may then be used to compute the bioimpedance Z according to the well known formula: $Z = U/I$.

The measurement circuitry 114 may further comprise or have access to at least one memory 220. The memory 220 may store a computer program code comprising instructions readable and executable by the processor(s) 204 and configuring the above-described operation of the processor(s). The memory 220 may further store a configuration database 224 defining parameters for the processor(s), e.g. parameters for the current feed control.

The apparatus may further comprise a communication circuitry configured to transmit measurement data acquired by the measurement circuitry to an external device such as a smart phone or a wrist computer. The external device may be a training computer configured to monitor a physical exercise performed by the user. The communication circuitry may be a wireless communication circuitry supporting a wireless communication protocol such as ANT, ANT+, or Bluetooth®, e.g. Bluetooth Smart ®.

From the measured bioimpedance, other physiological characteristics such as a heart stroke volume may be computed. Patent publication US 2002/0193689 discloses one method of computing the stroke volume by using the bioimpedance and the ECG, and the processor 204 may employ such a method in some embodiments.

In the embodiment of Figure 1, the electrodes 120, 122 may be used as both feed electrodes through which the electric current is applied and as voltage measurement electrodes. A switching mechanism may be applied which switches the function of the electrodes 120, 122 between the current feed and the voltage measurement. The switching mechanism may perform time-multiplexing and connect the electrodes either to the current generator 202 or the voltage measurement circuitry, under the control of the processor 204 and depending on the desired function of the electrodes 120, 122.

In a further embodiment, one or more of the electrodes 120 and 122 are further configured to measure the ECG. In such a case, the switching mechanism may further control switching of the electrodes to a differential amplifier used as a front-end in the ECG measurements.

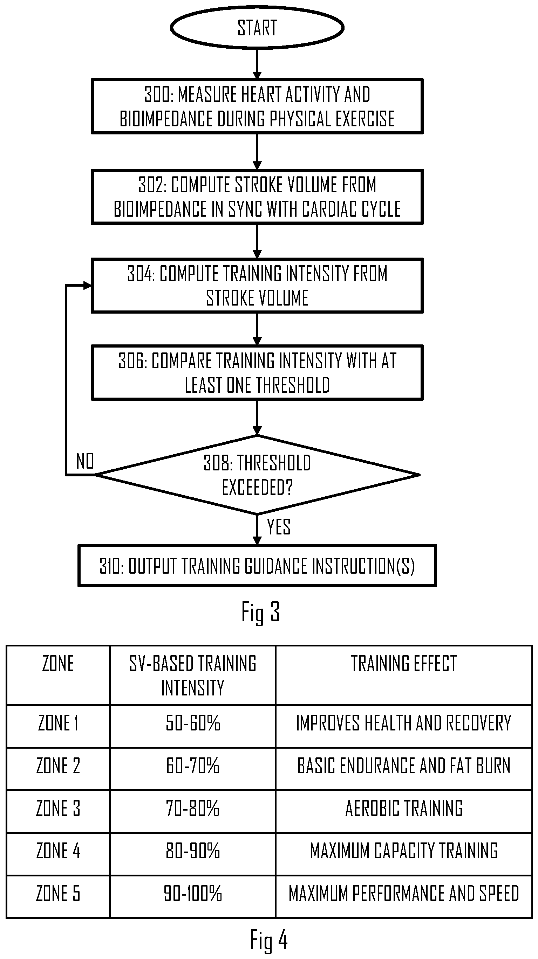

The above-described measurement configuration may be used in the following embodiments for monitoring a physical exercise performed by the user 100. Figure 3 illustrates a process for providing training guidance in connection with the physical exercise. The process may be executed by a training computer. Referring to Figure 3, the process comprising: measuring (block 300), by using a heart activity sensor, heart activity of a user during the physical exercise; measuring (block 300), by using a bioimpedance measurement sensor, bioimpedance of the user during the physical exercise; computing (block 302), by a processing circuitry, a stroke volume from the bioimpedance synchronized to a cardiac cycle of the user by using the measured heart activity; computing (block 304), by the processing circuitry, training intensity for the physical exercise or a part of the physical exercise by using at least the computed the stroke volume; and comparing (block 306 and 308), by the processing circuitry, the training intensity with at least one threshold and outputting (block 310) at least one training guidance instruction on the basis of the comparison.

Computation of the training intensity by using the stroke volume provides several advantages over conventional techniques that determine the training intensity from the heart rate. The heart rate does not yield the whole picture of cardiac output and user's effort level. This may lead for example to inaccurate training load or energy expenditure estimation. A typical example where the heart rate is a sub-optimal measure is high intensity interval training (HIIT) or strength training. After finishing a high-intensity work period, the heart rate drops relatively fast. This results in estimation of a mild training effect for the exercise although the user's muscles become exhausted. Stroke volume behaves differently and provides better correlation with tissue saturation index than the heart rate. The tissue saturation index is a measure of oxygenated haemoglobin in the blood and may be considered to represent true training intensity. The stroke volume may also increase after the heart rate has reached its maximum. It means that the estimation of the training intensity by using the stroke volume enables quantification of the training intensity when the heart rate has saturated. Accordingly, computation of the training intensity by using the stroke volume provides better accuracy in the estimation of the training intensity during and/or after the exercise.

In an embodiment, the training intensity is computed from the stroke volume.

In an embodiment, the processing circuitry computes heart rate from the measured heart activity, and computes the training intensity from cardiac output (CO) defined by a product of the computed stroke volume (SV) and the heart rate (HR) as:

$$CO(n) = SV(n) \times HR(n)$$

where n represents a time/sample index. As described above, the bioimpedance and the heart activity may be computed synchronously. The CO may thus be computed from samples or sample sets having the same time index or indices.

In an embodiment, the processing circuitry computes the heart rate from the measured heart activity, and further computes the training intensity from oxygen intake (VO2) defined by a product of the computed stroke volume, the heart rate, and a constant factor as:

$$VO2(n) = SV(n) \times HR(n) \times avdiff$$

where *avdiff* represents arteriovenous oxygen difference. *avdiff* is an indication of how much oxygen is removed from the blood in capillaries as the blood circulates in the body. In another words, it can be defined as a net difference of oxygen content between aorta and vein in terms of litres of oxygen per litre of blood. For the processing circuitry, this factor may be considered as a constant. It may be a predefiend user-specific parameter.

In an embodiment, the processing circuitry computes the heart rate from the measured heart activity, and further computes the training intensity from energy expenditure (EE) defined by a product of the computed stroke volume, the heart rate, the factor *avdiff,* and a predetermined user-related parameter as:

$$EE(n) = SV(n) \times HR(n) \times avdiff \times oec$$

where *oec* is an oxygen-to-energy coefficient that represents the user's ability to convert oxygen into energy, e.g. 5 kilocalories per litre of oxygen. EE may be a momentary energy expenditure at timing n.

Any one or a combination of the above-described training intensity measures may be used in block 304. All of them are training intensity metrics based on the stroke volume.

In an embodiment, the processing circuitry may utilize training intensity zones that are mapped to different training intensity ranges by using the stroke volume as a factor for the training intensity. In this embodiment, the processing circuitry may determine a plurality of training intensity zones on the basis of the stroke volume, wherein ranges of each training intensity zone is mapped to a unique range of stroke volume values. Thereafter, the processing circuitry may perform said comparison in blocks 306 and 308 by comparing the training intensity with at least one training intensity zone. In this embodiment, the at least one threshold may comprise at least one limit of the at least one training intensity zone.

The training intensity zones may be created for any one of the above-described training intensity measures, e.g. the SV, CO, VO2 or EE. All of them are based on the stroke volume and, thus represent the training intensity and training effect better than heart rate zones, for example. Another feature that distinguishes the stroke-volume-based training intensity zones from the heart rate zones, for example, is that the processing circuitry may indicate the zones to the user by using a different factor than that on which the zones are based, e.g. the stroke volume. The zone ranges may be mapped to the values of SV, CO, VO2, or EE but the training intensity zones may be indicated to the user by using verbal definitions or by percentages from the maximum value, as illustrated in the middle column of Figure 4. Stroke volume or cardiac output values as such may not be illustrative to an average user and, therefore, use of more illustrative zone definitions serves the purpose of improved training guidance.

Figure 4 illustrates an example set of training intensity zones that are based on the SV. Each zone may be associated with a unique range of the selected training intensity measure. The ranges of the zones in terms of the selected stroke-volume-based training intensity measure may be selected such that the training effect (right-most column) and associated training intensity (middle column) described in Figure 4 is satisfied. The relative training intensity ranges in the middle column are examples and different ranges may be applied.

Let us now describe an embodiment for determining at least some of the ranges of the training intensity zones. In this embodiment, the processing circuitry may be configured to carry out a process comprising: determining a lactate threshold of the user within a range of a training intensity; determining, on the basis of the lactate threshold, an aerobic threshold and an anaerobic threshold of the user within the range of the training intensity; measuring the stroke volume at the aerobic threshold and/or anaerobic threshold; and using the measured stroke volume at the aerobic threshold and anaerobic threshold as the at least one threshold, e.g. a limit of a stroke-volume-based training intensity zone.

For example, the lactate threshold may be used when mapping a determined heart rate or speed of motion of the user to the aerobic and anaerobic thresholds. This may be carried according to conventional means. The heart rate and the speed of motion are examples of the training intensity mentioned in the previous paragraph. Then, the processing circuitry may acquire the stroke volume for the determined heart rate or the speed of motion at the aerobic/anaerobic threshold, thus mapping the stroke volume to the respective thresholds. In a similar manner, the other training intensity measures CO, VO2 and EE may be mapped to the respective thresholds. Thereafter, the other stroke-volume-based training intensity zones may be created.

This procedure allows also determination of maximum values for the SV, CO, and VO2. The maximum value is then mapped to the upper limit of the training intensity zone associated with the highest training intensity. In an embodiment, the processing circuitry may determine the maximum value for the SV, CO, and VO2 on the basis of measurements carried out during the exercise and, optionally, other exercises. The processing circuitry may determine and store a sports-type-specific maximum value for any one of the training intensity measures. The stroke volume is a parameter that typically evolves according to the user's fitness and, therefore, it may be advantageous to update the maximum value regularly. For example, when the user is performing and exercise of a determined sports type, e.g. cycling, the processing circuitry may monitor a maximum value of the training intensity parameter measured during the exercise and compare the maximum value with a stored maximum value associated with the sports type. If a maximum value higher than the stored maximum value is measured during the exercise, the processing circuitry may update the stored maximum value with the one measured and, optionally, update ranges of the stroke-volume-based training intensity zones accordingly.

In a similar manner, the SV, CO, and VO2 values at rest may be determined and used as a minimum value for the respective parameter.

As described above, the processing circuitry may store different stroke-volume-based maximum values concurrently for different sports types.

The computation of the stroke volume may be carried out by using the measurement configuration of Figure 1. In other embodiments, further electrodes may be used to realize the bioimpedance measurements and the ECG measurements. There may be provided dedicated electrodes used as the current feed electrodes and further dedicated electrodes as the measurement electrodes for the bioimpedance measurement. Each electrode may be provided at a different location. The locations of the electrodes may be selected such that a line drawn from one measurement electrode to another measurement electrode intersects with a line drawn from one feed electrode to the other feed electrode. This provides symmetricity between the current feed and the voltage measurement. Further electrodes may be configured as ECG measurement electrodes and disposed at an arbitrary location in the garment.

In another embodiment, some of the electrodes are configured to function as both the voltage measurement electrodes for the bioimpedance measurements and as the ECG measurement electrodes by using the above-mentioned switching mechanism. In this embodiment as well, the feed electrodes and the measurement electrodes may be disposed such that the above-mentioned lines intersect.

In some embodiments, one of the feed electrodes is above the heart level while the other one of the feed electrodes is below the heart level. In a similar manner, one of the measurement electrodes is above the heart level while the other one of the measurement electrodes is below the heart level.

The garment may have a backside arranged to face a backside of the user and further have a front side arranged to face a front side of the user. In any one of the embodiments described herein, the electrode(s) disposed above the heart level may be disposed at a neck or shoulder area of the garment on at least the backside of the garment. This improves the skin contact during a physical exercise such as running. In an embodiment, the electrode(s) above the heart level is/are elongated and extend(s) from the backside of the garment to the front side of the garment over a shoulder of the user. During the exercise, the shoulder area of the garment typically has the best skin contact, and this embodiment further improves the skin contact.

The electrode(s) disposed below the heart level may be disposed in a chest area of the garment, wherein the garment is arranged to be form-fitting at the location of the second electrode. The form-fitting may be realized by the elastic material of the garment or by a strap in the garment.

In embodiments modified from those described above, at least some of the electrodes may be disposed on an opposite side of the human body. For example, one or more or even all the electrodes may be disposed on the back side of the body. Those electrodes disposed below the heart level may be disposed on the back side of the garment or on either side. In an embodiment, the garment comprises one or more electrodes disposed below the heart level on the back side and further electrode(s) disposed below the heart level on the front side.

In an embodiment, the feed electrodes may have a different shape than the measurement electrodes. For example, the measurement electrodes may be elongated while the feed electrodes may have a round or point shape. The point shape enables more accurate determination of the current path between the feed electrodes and, thus, simplifies the system configuration. Elongated measurement electrodes provide a better skin contact for the measurements and, thus, improved measurement accuracy.

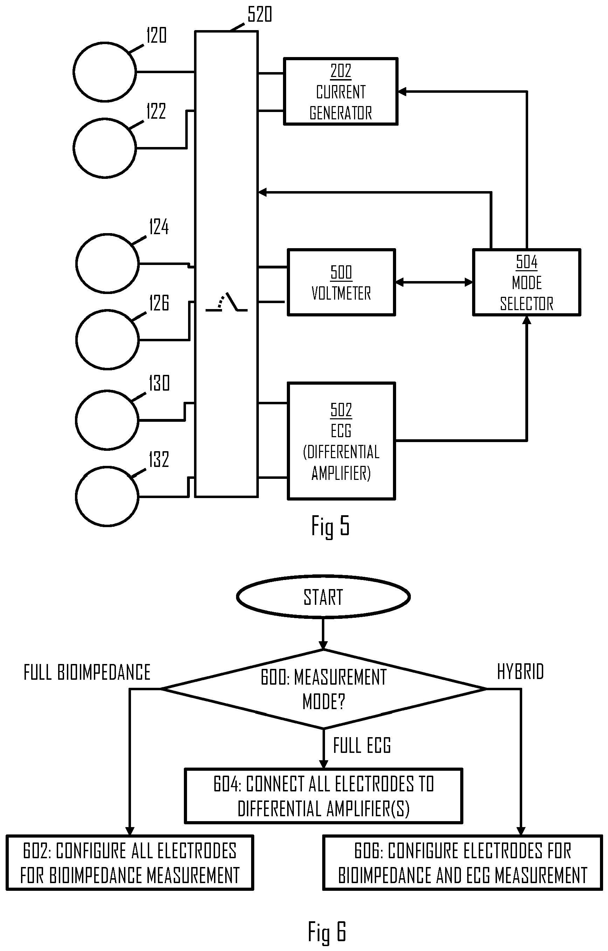

Figure 5 illustrates a block diagram of the measurement circuitry comprising the switching mechanism 520. The switching mechanism 520 may be realized by one or more switches that perform(s) switching a function of at least one of the measurement electrodes between the ECG measurement and bioimpedance measurement. The switching mechanism 520 may comprise one or more electronic switches.

In an embodiment the switching mechanism 520 switches the function of the measurement electrodes between at least two of the following measurement modes: full ECG mode where all measurement electrodes are used to measure ECG, a full bioimpedance measurement mode where all measurement electrodes are used to measure bioimpedance, and a hybrid measurement mode where a first subset of the measurement electrodes are used to measure ECG and a second subset of the measurement electrodes are used to measure bioimpedance. Electrodes 120 and 122 represent current feed electrodes for the bioimpedance measurements, electrodes 124 and 126 represent voltage measurement electrodes for the bioimpedance measurements, and electrodes 130 and 132 represent the ECG measurement electrodes. The electrodes of Figure 5 may be integrated into the garment. The switching mechanism may be controlled by the processor 204. The processor 204 may comprise a mode selector circuitry 502 configured to perform a process for selecting a measurement arrangement according to the flow diagram of Figure 6.

Referring to Figure 6, the mode selector circuitry 502 selects the measurement mode in block 600. The selection may be made based on a measurement profile acquired on the basis of a user input, for example. Whenever bioimpedance measurements are coupled by the switching mechanism 520, the current generator 202 is coupled to current feed electrodes 120, 122 and the voltage measurement circuitry 500 is coupled to the voltage measurement electrodes 124, 126. Whenever the ECG measurements are coupled by the switching mechanism 520, the ECG measurement circuitry 502 is coupled to ECG measurement electrodes 130, 132.

Upon selecting the full ECG mode in block 600, the process may proceed to block 604 where the mode selector configures the switching mechanism 520 to couple all the electrodes 120 to 132 of Figure 5 to inputs of an ECG measurement circuitry 502. The ECG measurement circuitry 502 may comprise a plurality of differential amplifiers, and each pair of electrodes 120 to 132 may be connected to inputs of one of the differential amplifier. For example, the switching mechanism 520 may couple the electrodes 120 and 122 to different inputs of one of the differential amplifiers, the electrodes 124 and 126 to different inputs of another one of the differential amplifiers, and the electrodes 130 and 132 to different inputs of yet another one of the differential amplifiers. In this manner, the switching mechanism may realize a multi-channel ECG measurement arrangement where two or more pairs of electrodes are connected to the ECG measurement circuitry 502. The mode selector 504 may further activate the ECG measurement circuitry 502 to start the multi-channel ECG measurements. The ECG measurement circuitry 502 may combine the ECG measurement signals received through the different measurement channels. Full ECG mode may be used when only the heart rate or heart activity is monitored, e.g. during a physical exercise.

Upon selecting the full bioimpedance mode in block 600, the process may proceed to block 602 where the mode selector 504 configures the switching mechanism 520 to couple all the electrodes 120 to 132 for bioimpedance measurements. In the full biompedance mode, the switching mechanism 520 may couple at least one pair of the electrodes for current feed and at least one pair of electrodes for voltage measurement. In an embodiment, the current feed electrodes may be coupled to a current generator 202 configured to feed constant current. In an embodiment, the voltage measurement electrodes are coupled to a voltmeter 500 configured to measure voltage between the voltage measurement electrodes while the current generator feeds the current.

In the embodiment using four electrodes, e.g. electrodes 120 to 126, electrodes 120 and 122 may be coupled to the current generator 202 for current feed and electrodes 124, 126 to the voltmeter 500, as described above in connection with Figure 3. In the embodiment using six electrodes, e.g. electrodes 120 to 132, a measurement channels may be realized by coupling one or more of the electrodes 130, 132 also to the voltmeter 500. For example, a further voltage measurement may be realized between electrodes 124 and 132 and/or 124 and 130.

In the embodiment using only two electrodes, the switching mechanism may be configured to alternately switch the electrodes to the current generator 202 and to the voltmeter 500 with a determined frequency. In this manner, only two electrodes may be used when measuring the bioimpedance. The voltmeter may be configured to measure a voltage sample while the electrodes are coupled to the voltmeter and not take samples while the electrodes are coupled to the current generator.

The full bioimpedance mode may be used when measuring body composition, for example. In the full bioimpedance mode, the current generator may be configured to output currency one at least two frequencies, either simultaneously or in a time-multiplexed manner.

Upon selecting the hybrid mode in block 600, the process may proceed to block 606 where the mode selector 504 configures the switching mechanism 520 to couple a subset of the electrodes for the bioimpedance measurements and another subset of electrodes for the ECG measurements. This mode may be employed when measuring the stroke volume and heart rate during a physical exercise or when measuring the body composition and the heart rate simultaneously, for example.

In the hybrid mode, the switching mechanism may couple at least two electrodes to the ECG measurement circuitry 502, at least two electrodes to the current generator 202, and at least two electrodes to the voltmeter 500. In the embodiment of Figure 3 using six electrodes, each electrode may be configured to a specific function in a fixed manner in the hybrid mode. For example, the electrodes 120, 122 may be coupled to the current generator 202, electrodes 124, 126 to the voltmeter 500, and electrodes 130, 132 to the ECG measurement circuitry 502.

In the embodiment using a reduced set of electrodes, e.g. four electrodes, the switching mechanism 520 may be configured to alternately switch the electrodes to the ECG measurement circuitry 502 and to the voltmeter 500 with a determined frequency. The voltmeter may be configured to measure a voltage sample while the electrodes are coupled to the voltmeter and not take samples while the electrodes are coupled to the ECG measurement circuitry. The ECG measurement circuitry 502 may be configured to measure an ECG sample while the electrodes are coupled to the ECG measurement circuitry and not take samples while the electrodes are coupled to the voltmeter.

In the embodiments using the alternating switching, the switching frequency may be higher than 60 Hertz (Hz).

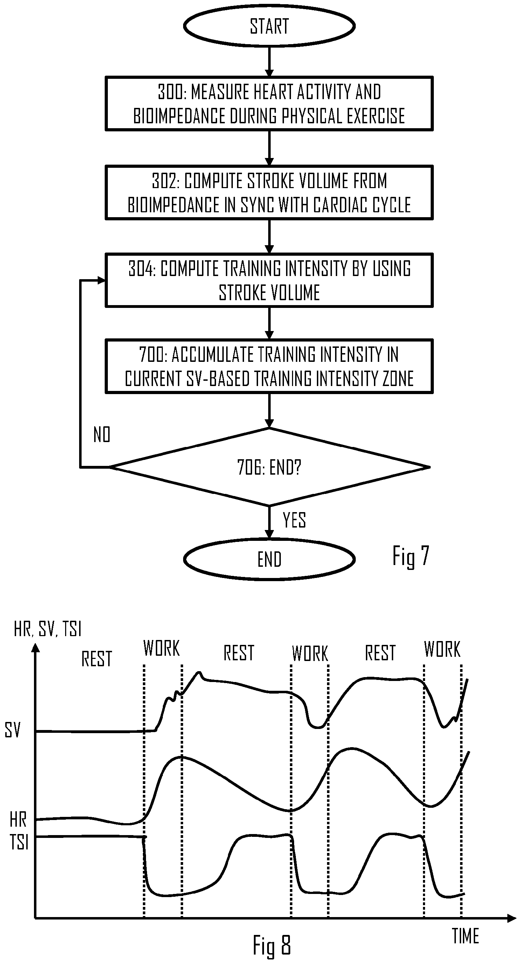

In an embodiment, the processing circuitry is configured to estimate a training load of the physical exercise by using the measured stroke volume. The training load may be estimated by accumulating the computed training intensity. The aggregate training intensity accumulated during the physical exercise represent the load of the exercise on the user. Figure 7 illustrates an embodiment for accumulating the training intensity. Referring to Figure 7, blocks 300 to 304 may be carried out in the above-described manner. The processing circuitry may compute the training intensity repeatedly during the physical exercise in block 304. In block 700 upon computing a training intensity value, the processing circuitry may determine a training intensity zone to which the training intensity value falls and accumulate the training intensity in the training intensity zone. The accumulation may comprise accumulating time spent by the user in the training intensity zone during the physical exercise. Upon accumulating, the processing circuitry may determine whether or not to compute the subsequent training intensity value (block 706). Upon determining the compute the subsequent training intensity value, the process returns to block 304. Otherwise, the process may end, e.g. at the end of the exercise.

In an embodiment, upon ending the accumulation, the processing circuitry may compute the training load of the physical exercise on the basis of results of said accumulating output the at least one training guidance instruction on the basis of the training load. The processing circuitry may determine the time spent on each training intensity zone and compute, on the basis of the times and respective intensities of the zones, the training load. The training load may be estimated in terms of recovery time the user needs to recover from the exercise.

As described above, the heart rate may be a sub-optimal metric for measuring the training intensity and training load of a strength training or HIIT exercise. Figure 8 illustrates exemplary curves of behaviour of the heart rate, stroke volume, and tissue saturation index (TSI) during an HIIT exercise which is an example of an interval exercise comprising work periods and rest periods. The user performs with high training intensity during the work periods and rests or performs with mild training intensity during the rest periods. In this example, the work periods are shorter than the rest periods. The general tendency with the TSI is that the TSI drops dramatically shortly after the start of the high-intensity work period and slowly recovers during the rest period. This indicates that the user works out in an anaerobic zone during the work period, which is typical for the HIIT exercise. The heart rate increases during the work period but falls quite quickly during the rest period, depending on the user's heart rate recovery capability. However, the stroke volume rises during the work periods and remains high during the work period. Eventually, the SV starts to drop but at a much slower pace than the heart rate, for example.

HIIT is an efficient exercise to maximize time at maximal SV. The SV has been shown to remain high during rest periods or even surpass SV values measured during the work periods, while VO2 as well as HR decrease quite rapidly during the rest periods. Reducing the training intensity of the rest periods or even resting during the rest periods of the HIIT exercise may therefore prolong the time to exhaustion. It may also allow the accumulation of more time on high-intensity zones, prolong accumulated time spent at maximal SV, maximal CO, maximal VO2, and/or maximal EE and lead to improved training benefit.

In an embodiment, the processing circuitry determines the training guidance such that the SV values are maximized. The processing circuitry may instruct the user to perform to maintain the SV above a determined threshold level. In an embodiment, upon detecting that the SV drops below a determined level, the processing circuitry may instruct the user to increase the training intensity. Figure illustrates an embodiment where the processing circuitry adapts the work periods of the interval exercise to the observations of the measured stroke volume. In this embodiment, the at least one threshold comprises a threshold for triggering the next work interval after a rest period of the physical exercise

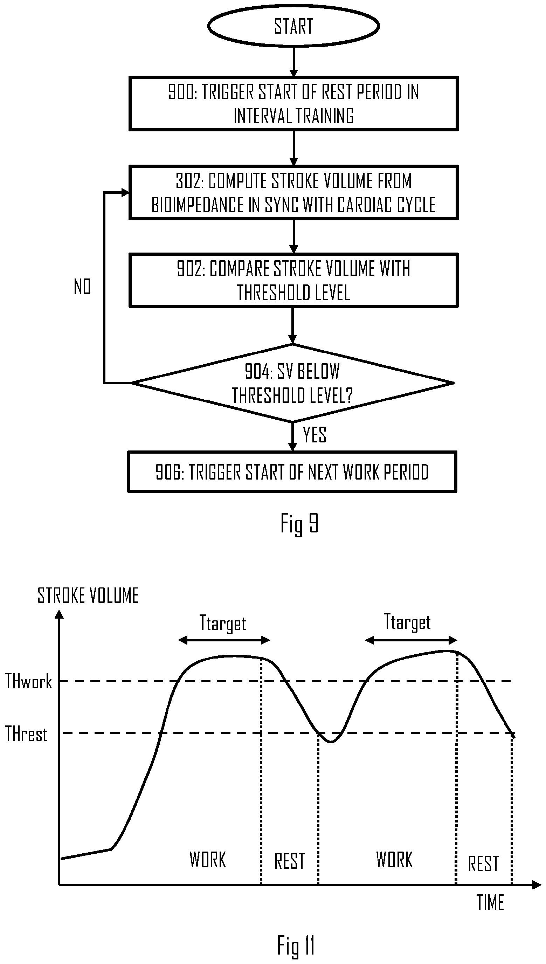

Referring to Figure 9, the processing circuitry may trigger the start of the rest period in block 900. The trigger may be the end of the previous work period, and the end may be detected on the basis of measured time of the work period or measured training intensity accumulation during the work period. During the rest period, the processing circuitry may execute block 302 and compute the SV. In block 902, the processing circuitry compares the computed stroke volume with the threshold for triggering the next work interval. Upon detecting in block 904 that the comparison indicates that the stroke volume has dropped below a determined level defined by the threshold (YES in block 904), the processing circuitry outputs the training guidance instruction that instructs the user to start the next work interval (block 906). Otherwise, the process may return to block 302 to compute the next SV value. This embodiment enables maintenance of the SV above the threshold level, thus optimizing the training effect of the interval training.

In an embodiment, the determined level defined by the threshold is a selected drop of the stroke volume from a reference stroke volume measured at the start of the recovery interval, e.g. a value between 5 and 15 percent. In other words, when the SV has dropped for an amount determined by the value from the start of the rest period, the processing circuitry may trigger the next work period.

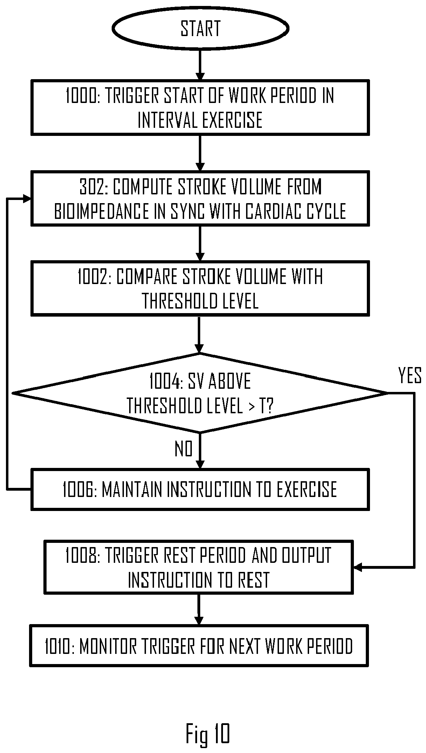

In an embodiment, a similar approach for adapting the length of the work period is utilized by the processing circuitry and, in particular, the end of the work period. In this embodiment, the at least one threshold comprises a threshold indicating a minimum training intensity for a work period of the interval exercise. The processing circuitry accumulates time the stroke volume remains above the threshold during the work period, and outputs the training guidance instruction as an instruction to end the work period. The instruction is triggered by the processing circuitry upon detecting that the stroke volume has remained above the threshold for a determined target time interval T. Figure 10 illustrates a process according to this embodiment.

Referring to Figure 10, the processing circuitry triggers the start of the work period in the interval exercise in block 1000. The start may be triggered according to the process of Figure 9 or by using a preset timing for the duration and end of the previous rest period. During the work period, the processing circuitry may compute the stroke volume in the above-described manner in block 302. The computed stroke volume may be compared with the threshold indicating the minimum training intensity for the work period in block 1002. If the stroke volume indicates training intensity above the threshold level, the processing circuitry may accumulate the time the training intensity is above the threshold level (not shown). In block 1004, the processing circuitry compares the accumulated time with the target time interval T. If it is determined in block 1004 that the training intensity indicated by the stroke volume has remained above the threshold level for the target time interval or above the target time interval, the process may proceed to block 1008 where the processing circuitry triggers the end of the work period, start of the subsequent rest period, and outputs an instruction for the user to end the work period. Thereafter, the processing circuitry may start monitoring for the trigger of starting the next work period in block 1010, e.g. according to the embodiment of Figure 9. Upon determining in block 1004 that the training intensity has not yet been above the threshold level long enough, the process may maintain the instruction to continue exercise (block 1006) and return to block 302.

Figure 11 illustrates an example of guidance during the exercise in the form of a curve illustrating a stroke-volume-based training intensity during the exercise. Y-axis of the graph of Figure 1 represents the stroke-volume-based training intensity such as the SV, CO, VO2, or EE. Two thresholds are mapped to the Y-axis, $TH_{work}$ for the accumulation of the training intensity during the work period according to the embodiment of Figure 10 and $TH_{rest}$ for triggering the end of the rest period according to the embodiment of Figure 9. Referring to Figure 11, the start of the interval exercise may be triggered in an arbitrary manner, e.g. the user starting the exercise and providing a start input to the training computer executing processes of Figures 9 and 10. Accordingly, the first work period starts. A warm-up phase may precede the first work period but that is omitted in this description for the sake of conciseness. During the first work period, the processing circuitry executes the process of Figure 10, computes the stroke volume and compares the SV-based training intensity with the threshold $TH_{work}$. Whenever the training intensity is above the threshold, a time value is accumulated. When the time value reaches the target time interval $T_{target}$, the processing circuitry triggers the start of the rest period and starts comparing the stroke volume with the threshold $TH_{rest}$. When the stroke volume drops below the threshold $TH_{rest}$, the processing circuitry may trigger the start of the next work period. In this manner, the procedure may continue until the end of the interval exercise.

In an embodiment, the SV-based training intensity monitored in the embodiment of Figure 9 may be different from the SV-based training intensity monitored in the embodiment of Figure 10. For example, any one of the SV-based training intensities (SV, CO, VO2, EE) may be suitable for embodiment of Figure 10 but it may be advantageous to measure only the SV in the embodiment of Figure 9. Let us remind that the HR may be sub-optimal for the adaptation of the rest period because of the characteristic of dropping quickly. Accordingly, metrics involving the HR may also be less optimal than a metric not having the HR but having the SV.

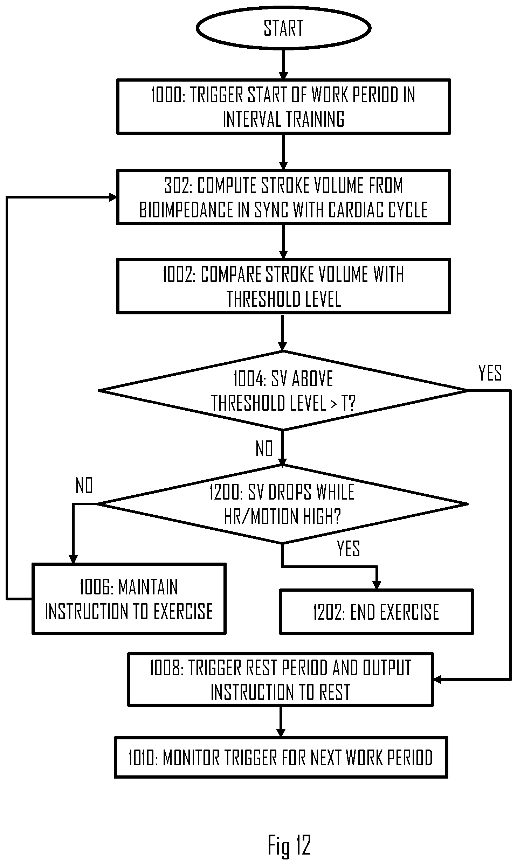

In an embodiment, the processing circuitry is configured to detect fatigue of the user from the computed stroke volume during the work interval and to output a training guidance instruction for the user to end the interval exercise. Figure 12 illustrates an embodiment for cancelling the exercise upon detecting that the user is fatigued. The fatigue can be detected by evaluating the SV and the heart rate during the work period. Referring to Figure 12, the processing circuitry may execute blocks 1000, 302, 1002, 1004, 1006, 1008, and 1010 in the above-described manner. Upon determining in block 1004 that the work period continues, the processing circuitry may next check the user's fatigue level. The checking may comprise evaluating the development of the SV. If the processing circuitry detects that the SV has dropped for at least a determined amount during the work period, e.g. by using threshold comparison, the processing circuitry may consider this as an indicator of fatigue and, next, check the development of the user's performance (block 1200). If one or more other indicators indicate that the user' is still performing with high intensity, the processing circuitry may in block 1200 determine that the user is fatigued and proceed to block 1202 where the processing circuitry ends the exercise. Block 1202 may comprise outputting an indication to the user to end the exercise. Block 1202 may comprise outputting an indication to the user that the user is possibly fatigued and should rest.

The one or more other indicators indicate that the user' is still performing with high intensity may comprise heart rate or motion intensity. The motion intensity may be measured by using a motion sensor, a force sensor, a cadence sensor, or a combination of these sensors. One or more thresholds may be employed in lock 1200, e.g. one for determining the sufficient drop in the SV and another for determining that the training intensity remains sufficiently high for triggering the end of the exercise.

Let us now return to the embodiments regarding the measurement configuration and hardware. The garment described above may be advantageous for accommodating the electrodes in the sense that the garment enables desired positioning of the electrodes with respect to the user's body. The measurement configuration may, however, be implemented by means other than the garment.

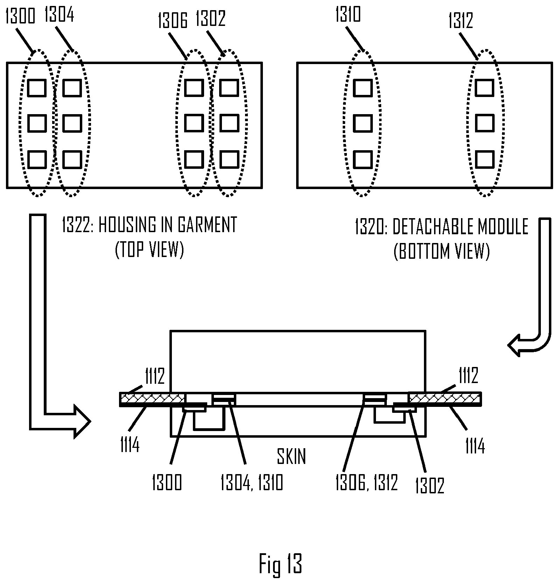

In an embodiment, a casing housing the electronics including the measurement circuitry 114 is detachable from the garment. The casing may be waterproof and attached mechanically to the garment by using snap fastening, for example. The snap fastening may also align the casing with respect to the garment such that the signal lines in the garment will couple with the corresponding interfaces in the casing. Figure 13 illustrates an example of such an embodiment.

Referring to Figure 13, the measurement circuitry may comprise a housing 1322 integrated or attached to the garment, e.g. in a fixed or permanent manner. The housing may function as a positioning member for the casing 1320 housing the measurement circuitry. The housing 1322 may comprise a first set of signal connectors 1300, 1302 that connect to the electrodes in the garment 1112, e.g. the ECG electrodes and bioimpedance electrodes. The housing 1322 may comprise a second set of signal connectors 1304, 1306 configured to provide connection with respect connectors in the casing 1320. The connectors 1304, 1306 may be exposed when the module 1320 is detached from the housing 1322. The connectors 1300, 1302 may be covered, e.g. in the housing, and connecting in the housing to the respective signal lines 1114 leading to the respective electrodes. Internal wiring may be disposed in the housing 1322 to connect the connectors of the first set 1300,1302 to the appropriate connectors of the second set 1304, 1306, as illustrated in the bottom of Figure 13.

The casing 1320 may comprise the set of connectors 1310, 1312 that are disposed such that the connectors connect to the appropriate connectors of the second set 1304, 1306 when the casing 1320 is attached to the housing. Internal wiring may be provided in the module to connect the connectors to respective components of the measurement circuitry, e.g. to the differential amplifier 502, voltmeter 500, and/or the current generator 202.

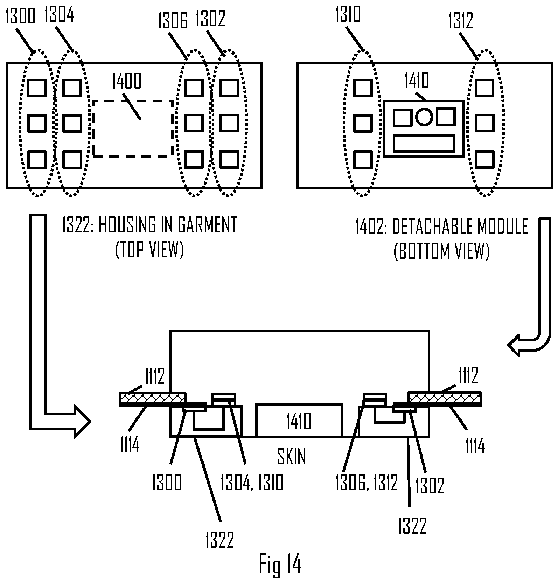

In an embodiment, the housing comprises a hole at the location where the casing is to be attached. Figure 14 illustrates such an embodiment. The hole 1400 is illustrated in the housing, and, in the corresponding location in the casing 1402, a sensor head 1410 is disposed. A sensor may be provided on a surface of the casing in the sensor head 1410 designed to enter the hole 1400 and contact the skin. The sensor head 1410 may comprise a photo sensor, a photplethysmogram (PPG) sensor, ECG sensor and/or a temperature sensor. In such embodiment, the sensor head 1410 in the casing may replace one or more of the above-described electrodes, depending on the location of the casing 1402 in the garment 1112. For example, if the location is above the heart level, the sensor head may replace one or more of the sensors 120, 124 and 134. If the location is below the heart level, the sensor head may replace one or more of the sensors 122, 126, 136. The sensor head may replace the ECG sensors 130, 132. The switching mechanism 520 may nevertheless work in the above-described manner.

Let us then return to the discussion of the embodiments regarding the training guidance and configuration of the processing circuitry. The embodiment of Figure 12 may be used also for detection of a dehydration state of the user.

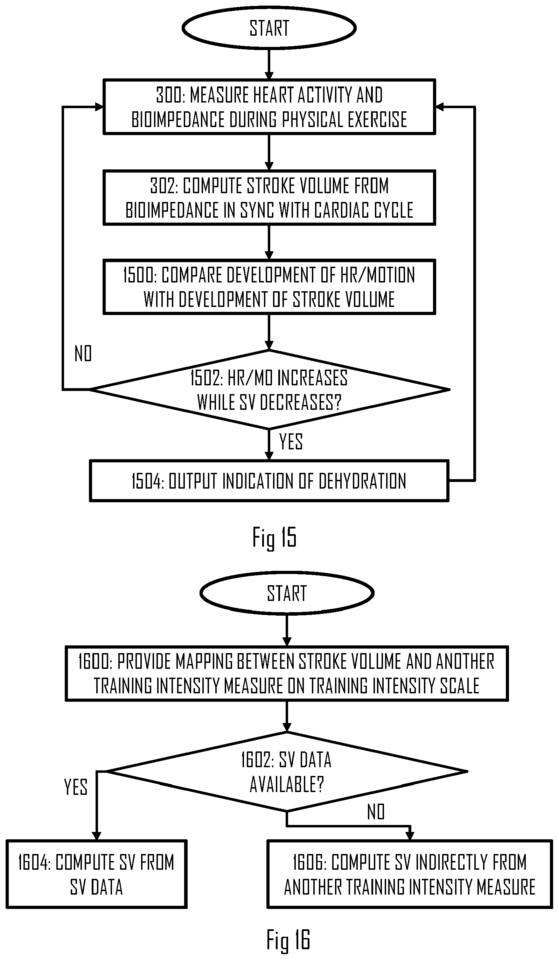

The dehydration may cause similar behaviour in the stroke volume as fatigue. However, it may be beneficial to monitor the dehydration also during the rest periods. Figure 15 illustrates an embodiment for monitoring the dehydration state of the user. Referring to Figure 15, the processing circuitry may carry out blocks 300 and 302 in the above-described manner. In block 1500, the processing circuitry performs the comparison in the development of the SV and the development of the heart rate (HR) and/or motion intensity. If the processing circuitry observes in block 1502 that the stroke volume has decreases by at least a determined amount while the training intensity based on the other indicator(s) such as the HR and/or motion intensity has not dropped, the process may proceed to block 1504 where the processing circuitry outputs an indication of the dehydration to the user. Otherwise, the process may return from block 1502 to block 300.

In order to distinguish the dehydration from the fatigue, the processing circuitry may employ further inputs such as body temperature measured from the user and/or environmental temperature and/or humidity. In an embodiment, the processing circuitry may adjust the thresholds used in block 1500 on the basis of the measured temperature and/or humidity. For example, when the environmental temperature is high and/or humidity is high, the thresholds may be adjusted such that the processing circuitry is more sensitive to the drop of the SV with respect to the observed training intensity in block 1502. In a similar manner, the processing circuitry may adapt the thresholds on the basis of the elapsed duration of the exercise. If the elapsed duration is high, e.g. at the end of a long exercise, the thresholds may be adjusted such that the processing circuitry is more sensitive to the drop of the SV with respect to the observed training intensity in block 1502. In the beginning of the exercise, the thresholds may be adjusted such that the processing circuitry is less sensitive to the drop of the SV with respect to the observed training intensity in block 1502. Another parameters for adapting the threshold may be fluid intake of the user. The user may provide an input indicating the amount of consumed liquids to the processing circuitry. Upon receiving such an input, the thresholds may be adjusted such that the processing circuitry is less sensitive to the drop of the SV with respect to the observed training intensity in block 1502.

In the embodiments where the processing circuitry performs both embodiments of Figures 12 and 15, the processing circuitry may utilize the overlapping computation efficiently. For example, the execution of block 1500 may be utilized for block 1200 to avoid double computation.

In an embodiment, the processing circuitry is configured to compute the SV even in a situation where the bioimpedance measurements are not available, e.g. the switching mechanism 520 has switched to the full ECG mode. Figure 16 illustrates an embodiment of a process for computing the stroke volume. Referring to Figure 16, the processing circuitry may provide a mapping table (block 1600), e.g. stored in a memory, where the mapping table maps stroke volume values with values of another training intensity measure such as the heart rate, speed of motion, power, acceleration, or force. The mapping table may be preset or acquired through measurements during previous exercises. As described above, the SV correlates with the training intensity, as does the other training intensity measures. This characteristic enables the provision of the mapping table. In block 1602, the processing circuitry determines, during the exercise, whether or not the SV measurement data is available. The SV measurement data may comprise the bioimpedance measurement data. If the SV measurement data is available, the SV may be computed in the above-described manner from the SV measurement data (block 1604). Block 1604 may be block 302, for example.

Upon detecting in block 1602 that the SV measurement data is not available, the processing circuitry may execute block 1606 where the SV is estimated by using measurement data of the other training intensity measure, e.g. the heart rate of motion, by using the mapping table. This enables estimation of the SV or any SV-based training intensity when the SV measurement data is not available during the physical exercise or during a certain moment of the exercise, e.g. during the full ECG mode.

In an embodiment, the measured stroke volume or cardiac output is used when estimating a performance index for the user. The performance index may be understood as a measure which compares achieved external load such as power or force with an internal effort level. A running index feature included in training computers of Polar Electro is an example of a performance index which is computed as a function of running speed and heart rate. The higher distance the user is capable of running faster and with lower heart rate is mapped to a higher running index. The running index may be used as an estimate of how long it takes for the user to run a marathon or a half marathon, for example.

Stroke volume measurements during exercise enable the computation of an improved performance index that takes the stroke volume into account, in addition to heart rate, when estimating the internal effort level.

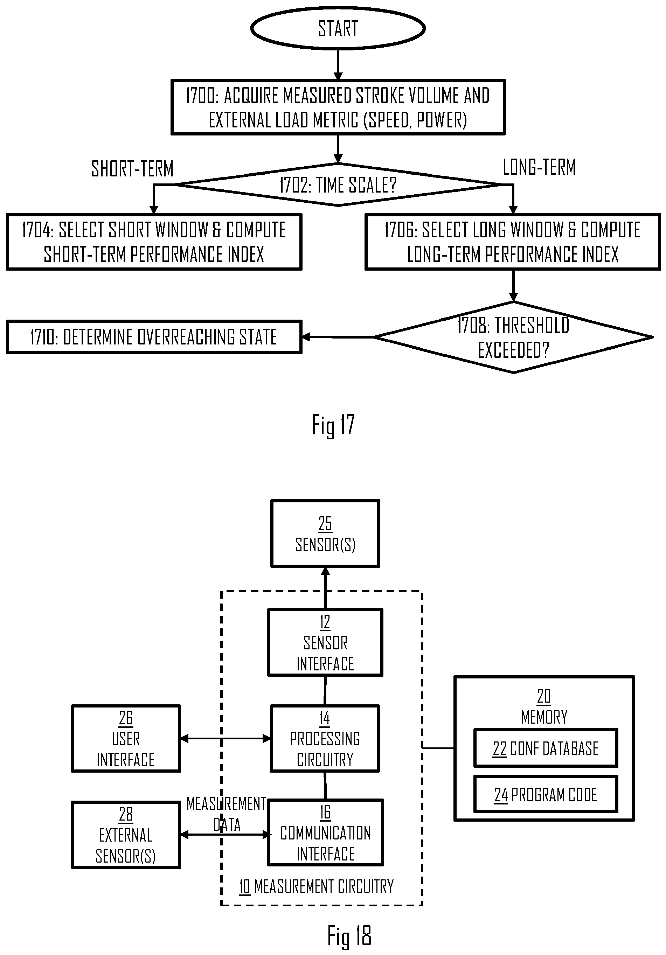

Figure 17 illustrates a process for estimating the performance index on the basis of the stroke volume measurements. The process may be executed as a computer-implemented process. Referring to Figure 17, the process comprises acquiring the measured stroke volume, e.g. according to any one of the above-described embodiments. Additionally, another measurement metric representing the external load such as speed, power, etc. measured during the exercise is acquired in block 1700. The speed may be measured by using a motion sensor and/or a (satellite) positioning receiver, for example. The power may be measured by using a power meter such as a strain gauge, for example. Other metrics are equally possible. In block 1702, the time scale of the performance index is determined. Short-term performance index may illustrate a different type of performance than a long-term performance index, as described below. Block 1702 may be executed before block 1702. For example, when the training computer is configured to start measurements for a physical exercise, the training computer may select the short-term mode. The training computer may perform the long-term performance index estimation over a longer time period, e.g. days, weeks, or months.

When the short-term performance index is selected in block 1702, the process proceeds to block 1704 where a short observation window is selected for the performance index estimation. The length of the window may be less than one minute, between one and five minutes, or between one and ten minutes, for example. In an embodiment, block 1704 is computed only during a physical exercise, i.e. when the training computer is configured to perform stroke volume measurements in a measurement mode associated with the exercise.

The computation of the performance index may follow the same principle as the running index described above. The performance index may be computed as a function or ratio of the external load (e.g. the speed) and the stroke volume. The measured heart rate may be used as an additional parameter. A greater external load (e.g. speed) output by the user with a lower stroke volume (and lower heart rate) is mapped to a higher performance index. The scale of the performance index may range from 1 to 10 or 1 to 100, for example, the higher value indicating the higher performance index and higher physical state of the user.

The performance index computed by using the short observation window, i.e. the stroke volume and external load measurements carried out within the observation window, indicates momentary efficiency of the user. Loss of efficiency (higher stroke volume ort cardiac output needed to produce the same power) may indicate fatigue or sub-optimal technique. Monitoring the performance index during the exercise may thus help the user in finding an optimal stride length in running. In other sports, the performance index may be used, for example, for finding optimal technique or style skiing or finding the right cadence when cycling. Monitoring the short-term performance index helps the user also in finding the exercise intensity that maximizes stroke volume, which helps to optimize training targeted at increasing maximal cardiac performance.

When the long-term performance index is selected in block 1702, the process proceeds to block 1706 where a long observation window is selected for the performance index estimation. The length of the window may be higher than duration of a single physical exercise, one or more days, one or more weeks, or one or more months. The window may span over multiple physical exercises such that the performance index will represent user's overall performance level. The performance index may be computed in the above-described manner, only the observation window is much longer than in block 1704.

In an embodiment, only a subset of measurement data available for the observation window is selected for the performance index estimation. The process may comprise determining measurement data acquired during standard conditions and selecting only such measurement data for the performance index computation. In block 1704, such standard conditions may be defined in terms of training intensity: only measurement data acquired when the training intensity is within a determined range. The training intensity may be determined according to any one of the above-described embodiments. In block 1706, such standard conditions may be determined in terms of the external load exceeding a certain threshold for at least a determined time interval or a determined period in training history. One or more exercises in the training history that do not qualify for the performance index estimation may be excluded, e.g. exercised performed when the user was sick.

In an embodiment, the performance index is computed as an amount of external load (e.g. speed) output by the user per CO.

In an embodiment, the performance index is computed as the stroke volume at a determined external load (e.g. speed) output by the user and, optionally, at a determined heart rate. The standard conditions may thus be determined as the determined external load and the determined heart rate. Accordingly, the measurement samples for the computation of the performance index may be selected to comprise exclusively the samples that meet the standard conditions.

In an embodiment, the long-term performance index is computed daily. In an embodiment, the long-term performance index is computed after or at the end of an exercise.

In an embodiment, the long-term performance index is computed by using the SV at rest. The rest state may be determined from the heart rate, e.g. the heart rate remains below a determined threshold, and/or from motion measurement data, e.g. the user is detected to stay still (sitting or lying down).

The long-term performance index may be combined with training history data, and overreaching or overtraining training status is recognized as a result of the combining. This is illustrated in Figure 17 in blocks 1708 and 1710. If the long-term performance index shows a decrease below a threshold for at least a determined period of time, e.g. the process may proceed to block 1710 to evaluate training load levels of the exercises the user has performed in the history. If the user's current training load level is higher than usual, the process may output a notification that the user is overreaching. The training load being higher than usual may be determine by setting a threshold level on the basis of the training load levels in the history. The threshold level may be computed by accounting only those training load levels that indicate high training load. The point in the threshold setting may be to determine the typical training load level at which the user has been stressed with the training load but still being able to recover normally from the stress. The threshold level may be computed as an average of those training load levels in the user's training history that represent training load above a determined level, e.g. a determined level of excess post-exercise oxygen consumption (EPOC). The EPOC is one measure of the training load and reflects recovery demands after an exercise. Formally expressed, it is the volume of excess post-exercise oxygen consumed reported in liters or ml/kg.

Figure 18 illustrates an embodiment of the training computer comprising the processing circuitry for carrying out the process of Figure 3 or any one of its embodiments in a computer-implemented process. The training computer may be a smart phone, a tablet computer, a wrist computer, or even a server computer. The training computer may comprise a measurement circuitry 10 configured to perform computation and interfacing when monitoring the physical exercise performed by the user, e.g. during or after the exercise. The measurement circuitry 10 comprises the above-described processing circuitry 14. The measurement circuitry may further comprise a sensor interface 12 configured to provide a communication connection with one or more sensors 25 internal to the training computer. For example, a wrist computer may comprise an ECG sensor or a PPG sensor for measuring the heart activity. In some embodiments, the sensor interface and the internal sensors are omitted. The measurement circuitry may further comprise a communication interface 16 providing wireless communication connection with the external sensors 28, e.g. with the measurement circuitry 114 comprised in the garment. The communication interface 16 may support Bluetooth® protocol, for example Bluetooth Low Energy or Bluetooth Smart.

The training computer may further comprise a user interface 26 comprising a display screen and input means such as buttons or a touch-sensitive display. The processing circuitry 14 may output the instructions regarding the exercise to the user interface 26.

The training computer may further comprise or have access to at least one memory 20. The memory 20 may store a computer program code 24 comprising instructions readable and executable by the processing circuitry 14 and configuring the above-described operation of the processing circuitry 14. The memory 20 may further store a configuration database 22 defining parameters for the processing circuitry, e.g. the thresholds and/or the mapping table of the embodiment of Figure 16.

As used in this application, the term 'circuitry' refers to all of the following: (a) hardware-only circuit implementations, such as implementations in only analog and/or digital circuitry, and (b) combinations of circuits and software (and/or firmware), such as (as applicable): (i) a combination of processor(s) or (ii) portions of processor(s)/software including digital signal processor(s), software, and memory(ies) that work together to cause an apparatus to perform various functions, and (c) circuits, such as a microprocessor(s) or a portion of a microprocessor(s), that require software or firmware for operation, even if the software or firmware is not physically present. This definition of 'circuitry' applies to all uses of this term in this application. As a further example, as used in this application, the term 'circuitry' would also cover an implementation of merely a processor (or multiple processors) or a portion of a processor and its (or their) accompanying software and/or firmware.

The techniques and methods described herein may be implemented by various means. For example, these techniques may be implemented in hardware (one or more devices), firmware (one or more devices), software (one or more modules), or combinations thereof. For a hardware implementation, the apparatus (es) of embodiments may be implemented within one or more application-specific integrated circuits (ASICs), digital signal processors (DSPs), digital signal processing devices (DSPDs), programmable logic devices (PLDs), field programmable gate arrays (FPGAs), graphics processing units (GPUs), processors, controllers, micro-controllers, microprocessors, other electronic units designed to perform the functions described herein, or a combination thereof. For firmware or software, the implementation can be carried out through modules of at least one chipset (e.g. procedures, functions, and so on) that perform the functions described herein. The software codes may be stored in a memory unit and executed by processors. The memory unit may be implemented within the processor or externally to the processor. In the latter case, it can be communicatively coupled to the processor via various means, as is known in the art. Additionally, the components of the systems described herein may be rearranged and/or complemented by additional components in order to facilitate the achievements of the various aspects, etc., described with regard thereto, and they are not limited to the precise configurations set forth in the given figures, as will be appreciated by one skilled in the art.

It will be obvious to a person skilled in the art that, as the technology advances, the inventive concept can be implemented in various ways. The invention and its embodiments are not limited to the examples described above but may vary within the scope of the claims.

**Claims**

1. A computer-implemented method for providing training guidance in connection with a physical exercise, comprising:

   measuring, by using a heart activity sensor, heart activity of a user during a physical exercise;
   measuring, by using a bioimpedance measurement sensor, bioimpedance of the user during the physical exercise;
   computing, by a processing circuitry, a stroke volume from the bioimpedance synchronized to a cardiac cycle of the user by using the measured heart activity;

computing, by the processing circuitry, training intensity by using at least the computed the stroke volume;

comparing, by the processing circuitry, the training intensity with at least one threshold and outputting at least one training guidance instruction on the basis of the comparison.

2. The method of claim 1, further comprising:

determining, by the processing circuitry, a plurality of training intensity zones on the basis of the stroke volume, wherein ranges of each training intensity zone is mapped to a unique range of stroke volume values; and

performing said comparison by comparing the training intensity with at least one training intensity zone, wherein the at least one threshold comprises at least one limit of the at least one training intensity zone.

3. The method of claim 1 or 2, further comprising:

computing, by the processing circuitry, the training intensity repeatedly during the physical exercise;

accumulating, by the processing circuitry, time spent by the user in each of a plurality of training intensity zone;

compute, by the processing circuitry, a training load of the physical exercise on the basis of results of said accumulating; and

outputting the at least one training guidance instruction on the basis of the training load.

4. The method of any preceding claim, further comprising computing heart rate from the measured heart activity, wherein the training intensity is a computed cardiac output defined by a product of the computed stroke volume and the heart rate.

5. The method of any preceding claim, further comprising computing heart rate from the measured heart activity, wherein the training intensity is a computed oxygen intake defined by a product of the computed stroke volume, the heart rate, and a predetermined user-related parameter representing arteriovenous oxygen difference.

6. The method of any preceding claim, further comprising computing heart rate from the measured heart activity, wherein the training intensity is a computed energy expenditure defined by a product of the computed stroke volume, the heart rate, a first user-related parameter representing arteriovenous oxygen difference, and a second user-related parameter representing oxygen-to-energy coefficient of the user.

7. The method of any preceding claim, wherein the training guidance instruction is for interval training and the physical exercise is an interval exercise, the at least one threshold comprises a threshold for triggering the next work interval after a recovery interval of the physical exercise, the method further comprising:

comparing, by the processing circuitry during a recovery period of the interval exercise, the computed stroke volume with the threshold for triggering the next work interval; and

outputting the training guidance instruction that instructs the user to start the next work interval when the comparison indicates that the stroke volume has dropped below a determined level defined by the threshold.

8. The method of claim 7, wherein the determined level defined by the threshold is a selected drop of the stroke volume from a reference stroke volume measured at the start of the recovery interval, wherein the selected drop is a value between 5 and 15 percent.

9. The method of any preceding claim, wherein the training guidance instruction is for interval training and the physical exercise is an interval exercise, and wherein the at least one threshold comprises a threshold indicating a minimum training intensity for a work interval of the interval exercise, wherein the comparison comprises accumulating time the stroke volume remains above the threshold during the work interval, and wherein the training guidance instruction is an instruction to end the work interval, the instruction triggered by the processing circuitry upon detecting that the stroke volume has remained above the threshold for a determined target time interval.

10. The method of any preceding claim, wherein the training guidance instruction is for interval training and the physical exercise is an interval exercise, and wherein the at least one threshold comprises a threshold indicating a minimum training intensity for a work interval of the interval exercise, the method further comprising detecting, by the processing circuitry, fatigue of the user from the computed stroke volume during the work interval and outputting said training guidance instruction for the user to end the interval exercise.

11. The method of claim 10, further comprising measuring motion measurement data by using at least one motion sensor during the physical exercise and detecting, by the processing circuitry, the fatigue by using the motion measurement data.

12. The method of any preceding claim, further comprising by the processing circuitry:

computing heart rate from the measured heart activity;
detecting increase in the heart rate and decrease in the stroke volume during the physical exercise; and
outputting an indication of dehydration to the user.

13. The method of any preceding claim, further comprising by the processing circuitry:

determining a lactate threshold of the user within a range of the training intensity;
determining, on the basis of the lactate threshold, an aerobic threshold and an anaerobic threshold of the user within the range of the training intensity; and
measuring the stroke volume at the aerobic threshold and anaerobic threshold; and
using the measured stroke volume at the aerobic threshold and anaerobic threshold as the at least one threshold.

14. An apparatus comprising means for carrying out all the steps of the method according to any preceding claim.

15. A computer program product readable by a computer and comprising program instructions which, when executed by the computer, cause the computer to carry out a computer process implementing all the steps of the method according to any preceding claim 1 to 13.

Fig 1

Fig 2

START

300: MEASURE HEART ACTIVITY AND BIOIMPEDANCE DURING PHYSICAL EXERCISE

302: COMPUTE STROKE VOLUME FROM BIOIMPEDANCE IN SYNC WITH CARDIAC CYCLE

304: COMPUTE TRAINING INTENSITY FROM STROKE VOLUME

306: COMPARE TRAINING INTENSITY WITH AT LEAST ONE THRESHOLD

308: THRESHOLD EXCEEDED?

NO

YES

310: OUTPUT TRAINING GUIDANCE INSTRUCTION(S)

Fig 3

| ZONE | SV-BASED TRAINING INTENSITY | TRAINING EFFECT |
|---|---|---|
| ZONE 1 | 50-60% | IMPROVES HEALTH AND RECOVERY |
| ZONE 2 | 60-70% | BASIC ENDURANCE AND FAT BURN |
| ZONE 3 | 70-80% | AEROBIC TRAINING |
| ZONE 4 | 80-90% | MAXIMUM CAPACITY TRAINING |
| ZONE 5 | 90-100% | MAXIMUM PERFORMANCE AND SPEED |

Fig 4

520

120

122

124

126

130

132

202
CURRENT
GENERATOR

500
VOLTMETER

504
MODE
SELECTOR

502
ECG
(DIFFERENTIAL
AMPLIFIER)

Fig 5

START

FULL BIOIMPEDANCE

600: MEASUREMENT
MODE?

HYBRID

FULL ECG

604: CONNECT ALL ELECTRODES TO
DIFFERENTIAL AMPLIFIER(S)

602: CONFIGURE ALL ELECTRODES
FOR BIOIMPEDANCE MEASUREMENT

606: CONFIGURE ELECTRODES FOR
BIOIMPEDANCE AND ECG MEASUREMENT

Fig 6

START

300: MEASURE HEART ACTIVITY AND
BIOIMPEDANCE DURING PHYSICAL EXERCISE

302: COMPUTE STROKE VOLUME FROM
BIOIMPEDANCE IN SYNC WITH CARDIAC CYCLE

304: COMPUTE TRAINING INTENSITY BY USING
STROKE VOLUME

700: ACCUMULATE TRAINING INTENSITY IN
CURRENT SV-BASED TRAINING INTENSITY ZONE

NO

706: END?

YES

END

Fig 7

HR, SV, TSI

REST   WORK   REST   WORK   REST   WORK

SV

HR
TSI

TIME

Fig 8

START

900: TRIGGER START OF REST PERIOD IN INTERVAL TRAINING

302: COMPUTE STROKE VOLUME FROM BIOIMPEDANCE IN SYNC WITH CARDIAC CYCLE

902: COMPARE STROKE VOLUME WITH THRESHOLD LEVEL

904: SV BELOW THRESHOLD LEVEL?

NO

YES

906: TRIGGER START OF NEXT WORK PERIOD

Fig 9

STROKE VOLUME

Ttarget

Ttarget

THwork

THrest

WORK

REST

WORK

REST

TIME

Fig 11

START

1000: TRIGGER START OF WORK PERIOD IN INTERVAL EXERCISE

302: COMPUTE STROKE VOLUME FROM BIOIMPEDANCE IN SYNC WITH CARDIAC CYCLE

1002: COMPARE STROKE VOLUME WITH THRESHOLD LEVEL

1004: SV ABOVE THRESHOLD LEVEL > T?

YES

NO

1006: MAINTAIN INSTRUCTION TO EXERCISE

1008: TRIGGER REST PERIOD AND OUTPUT INSTRUCTION TO REST

1010: MONITOR TRIGGER FOR NEXT WORK PERIOD

Fig 10

START

1000: TRIGGER START OF WORK PERIOD IN INTERVAL TRAINING

302: COMPUTE STROKE VOLUME FROM BIOIMPEDANCE IN SYNC WITH CARDIAC CYCLE

1002: COMPARE STROKE VOLUME WITH THRESHOLD LEVEL

1004: SV ABOVE THRESHOLD LEVEL > T?

YES

NO

1200: SV DROPS WHILE HR/MOTION HIGH?

NO

YES

1006: MAINTAIN INSTRUCTION TO EXERCISE

1202: END EXERCISE

1008: TRIGGER REST PERIOD AND OUTPUT INSTRUCTION TO REST

1010: MONITOR TRIGGER FOR NEXT WORK PERIOD

Fig 12

1300 1304    1306 1302        1310        1312

1322: HOUSING IN GARMENT        1320: DETACHABLE MODULE
      (TOP VIEW)                      (BOTTOM VIEW)

1112                                          1112

1114                                          1114

SKIN

1300        1304, 1310        1306, 1312        1302

Fig 13

1300 1304      1306 1302      1310      1312

1400      1410

1322: HOUSING IN GARMENT
(TOP VIEW)

1402: DETACHABLE MODULE
(BOTTOM VIEW)

1112      1112

1410

1114      SKIN      1114

1300      1304, 1310      1306, 1312      1302

1322      1322

Fig 14

START

**300: MEASURE HEART ACTIVITY AND BIOIMPEDANCE DURING PHYSICAL EXERCISE**

**302: COMPUTE STROKE VOLUME FROM BIOIMPEDANCE IN SYNC WITH CARDIAC CYCLE**

**1500: COMPARE DEVELOPMENT OF HR/MOTION WITH DEVELOPMENT OF STROKE VOLUME**

NO

**1502: HR/MO INCREASES WHILE SV DECREASES?**

YES

**1504: OUTPUT INDICATION OF DEHYDRATION**

Fig 15

START

**1600: PROVIDE MAPPING BETWEEN STROKE VOLUME AND ANOTHER TRAINING INTENSITY MEASURE ON TRAINING INTENSITY SCALE**

**1602: SV DATA AVAILABLE?**

YES

NO

**1604: COMPUTE SV FROM SV DATA**

**1606: COMPUTE SV INDIRECTLY FROM ANOTHER TRAINING INTENSITY MEASURE**

Fig 16

START

1700: ACQUIRE MEASURED STROKE VOLUME AND
EXTERNAL LOAD METRIC (SPEED, POWER)

SHORT-TERM          1702: TIME SCALE?          LONG-TERM

1704: SELECT SHORT WINDOW & COMPUTE
SHORT-TERM PERFORMANCE INDEX

1706: SELECT LONG WINDOW & COMPUTE
LONG-TERM PERFORMANCE INDEX

1710: DETERMINE OVERREACHING STATE          1708: THRESHOLD
EXCEEDED?

Fig 17

25
SENSOR(S)

12
SENSOR
INTERFACE

26
USER
INTERFACE

14
PROCESSING
CIRCUITRY

20
MEMORY

22 CONF DATABASE

24 PROGRAM CODE

MEASUREMENT
DATA

28
EXTERNAL
SENSOR(S)

16
COMMUNICATION
INTERFACE

10 MEASUREMENT CIRCUITRY

Fig 18

Europäisches
Patentamt

European
Patent Office

Office européen
des brevets

## EUROPEAN SEARCH REPORT

Application Number

EP 18 17 6971

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2012/035021 A1 (SAALASTI SAMI [FI] ET AL) 9 February 2012 (2012-02-09) * abstract * * paragraph [0026] * * paragraph [0048] - paragraph [0049] * * paragraph [0065] * * paragraph [0094] * * paragraph [0100] * * paragraph [0110] - paragraph [0122] * * paragraph [0147] * * figure 10 * | 1-15 | INV. A61B5/0205 A61B5/024 A61B5/0245 A61B5/029 A61B5/053 A61B5/11 A61B5/22 A61B5/00 |
| Y | US 2005/124901 A1 (MISCZYNSKI DALE J [US] ET AL) 9 June 2005 (2005-06-09) * paragraph [0002] * * paragraph [0066] * * paragraph [0194] * * paragraph [0204] * * figure 4 * | 1-15 | |
| A | US 2015/165271 A1 (LIN SUNG-LIEN [TW] ET AL) 18 June 2015 (2015-06-18) * paragraph [0001] * * paragraph [0020] - paragraph [0025] * | 1-15 | TECHNICAL FIELDS SEARCHED (IPC) A61B A63B |
| A | US 2015/031970 A1 (LAIN DAVID [US]) 29 January 2015 (2015-01-29) * paragraph [0015] - paragraph [0017] * * paragraph [0025] * * paragraph [0034] - paragraph [0039] * * paragraph [0045] * * paragraph [0050] * | 1-15 | |

The present search report has been drawn up for all claims

| Place of search | Date of completion of the search | Examiner |
|---|---|---|
| The Hague | 2 October 2018 | Gooding Arango, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 18 17 6971

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2018

| Patent document cited in search report | | Publication date | Patent family member(s) | | Publication date |
|---|---|---|---|---|---|
| US 2012035021 | A1 | 09-02-2012 | EP | 2035098 A1 | 18-03-2009 |
| | | | US | 2010216601 A1 | 26-08-2010 |
| | | | US | 2012035021 A1 | 09-02-2012 |
| | | | WO | 2008003830 A1 | 10-01-2008 |
| US 2005124901 | A1 | 09-06-2005 | NONE | | |
| US 2015165271 | A1 | 18-06-2015 | CN | 104706341 A | 17-06-2015 |
| | | | EP | 2897067 A1 | 22-07-2015 |
| | | | TW | 201521832 A | 16-06-2015 |
| | | | US | 2015165271 A1 | 18-06-2015 |
| US 2015031970 | A1 | 29-01-2015 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20020193689 A